# EUROPEAN PATENT APPLICATION

(11) **EP 4 450 089 A1**
(43) Date of publication of application: **23.10.2024**
(21) Application number: 22932930.5
(22) Date of filing: 01.07.2022
(51) Int. Cl.: A61K 41/00, A61K 9/70, A61K 47/42, A61P 35/00, A61K 49/22, A61K 49/00, A61M 37/00

(54) **MICRONEEDLE PATCH FOR ENHANCING ACCUMULATION OF PROTOPORPHYRIN IX IN SOLID TUMOR AND PREPARATION METHOD THEREFOR**

(30) Priority: 24.03.2022 CN 202210293369
(71) Applicant: Shenzhen University, Shenzhen, Guandong 518060 (CN)
(72) Inventor: HUANG, Peng, Shenzhen, Guangdong 518060 (CN); HE, Gang, Shenzhen, Guangdong 518060 (CN); LI, Yashi, Shenzhen, Guangdong 518060 (CN); LIN, Jing, Shenzhen, Guangdong 518060 (CN)
(74) Representative: KIPA AB
(86) International application number: PCT/CN2022/103330
(87) International publication number: WO 2023/178878

(57) **Abstract**

A microneedle patch for enhancing an accumulation of protoporphyrin IX in a solid tumor and a preparation method therefor. The method comprises the steps of: dissolving albumin in a Dulbecco's culture medium, using the albumin as a template, and adding CuCl2 and CaCl₂ for mineralization to obtain a CCP nanoparticle; by means of a one-step carbodiimide coupling method and an electrostatic adsorption method, loading CAT and 5-ALA to the surface of the CCP nanoparticle, respectively, to obtain a CCPCA nanoparticle; and loading the CCPCA nanoparticle and a microneedle matrix solution in a microneedle mold, drying under vacuum, and demolding to obtain a microneedle patch for enhancing an accumulation of PpIX in a solid tumor. According to the microneedle patch, by means of an integrated nano calcium phosphate carrier and a transdermal administration mode, an accurate delivery of CAT and 5-ALA is achieved, and PpIX and O2 levels in a tumor cell are increased. Meanwhile, the guidance of real-time bimodal PpIX fluorescence imaging and photoacoustic blood oxygen imaging is provided, and the photodynamic therapeutic effect on different cancers is improved.

## Description

### FIELD OF THE DISCLOSURE

The present disclosure relates to the technical field of microneedle-patch preparation and biomedicine, in particular to a microneedle patch for enhancing accumulation of Protoporphyrin IX in a solid tumor and a preparation method therefor.

### BACKGROUND

5-aminolevulinic acid (5-ALA) is selectively taken up by a tumor cell and rapidly biosynthesized into Protoporphyrin IX (PpIX) with fluorescence performance, which can be used for tumor diagnosis mediated by fluorescence imaging. Therefore, a photochemical reaction of 5-ALA-based photodynamic therapy (5-ALA-PDT) mainly occurs in a cancer cell, avoiding off-target toxic side effects. Although local 5-ALA-PDT becomes main treatment for patients with actinic keratosis or basal cell carcinoma, characteristics of limited intratumoral biosynthetic concentration of PpIX, short tumor-retention duration, tumor heterogeneity, tumor hypoxia microenvironment, and strong antioxidant system, etc. limit clinical applications of 5-ALA-PDT in solid-malignant-tumor treatment. In addition, in practical clinical applications, therapeutic plans of 5-ALA-PDT currently lack guidance of non-invasive molecular imaging, such as a time point of maximum intratumoral PpIX concentration, an intratumoral oxygen concentration, the number of times of laser irradiation, and other parameters. Therefore, there is an urgent need to develop more effective precise diagnosis-and-treatment solutions to solve clinical bottlenecks of 5-ALA-PDT, so as to improve biosafety of 5-ALA-PDT and further promote the clinical applications of 5-ALA-PDT.

Efficient intratumoral generation of PpIX is an important condition for 5-ALA-PDT, and high polarity and hydrophilicity of 5-ALA limit its passive diffusion across a stratum corneum and a cell membrane, leading to very-low bioavailability of 5-ALA in oral and transdermal administration. In another aspect, efficiency of converting 5-ALA taken up by a tumor cell to PpIX is critical, because the efficiency determines both highest concentration of PpIX generated intratumorally and tumor-retention duration. Due to Warburg effect of tumor-cell respiration, a respiratory dysfunction of the tumor cell leads to an imbalance in supply of PpIX and Fe²⁺. Studies show that biosynthesis of PpIX in the tumor cell may be upregulated by supplying exogenous 5-ALA, introducing iron chelator, reducing ferrochelatase expression, inhibiting heme-oxygenase activity, and other strategies. However, rapid increase of concentration of intracellular PpIX triggers a feedback regulation mechanism of heme synthesis, resulting in enhanced excretion of PpIX which used for the heme synthesis, then resulting in insufficient accumulated concentration of the intracellular PpIX, thus failing to achieve a desired tumor-treating effect. Studies show that Ras/MEK signaling pathway used for inhibiting proto-oncogene can reduce activity of ferrochelatase (FECH) by downregulating hypoxia-inducible factor (HIF)-1α (HIF-1α), thereby enhancing accumulation of intracellular PpIX. Therefore, continuous supply of oxygen (O₂) to the tumor cell can increase the accumulated concentration of intracellular PpIX by regulating the Ras/MEK signaling pathway and by cascading downregulating HIF-1α and FECH, and the problem of tumor hypoxia of 5-ALA-PDT can be overcame at the same time. However, inhibition of HIF-1α expression takes a relatively long duration, and metabolism of PpIX in vivo is relatively fast (a highest peak of synthesis is reached within about 4 hours, and a rapid clearance is within 12 hours), thus effective strategies are needed to prolong the tumor-retention duration of PpIX. A microneedle delivery system can directly penetrate a stratum corneum for transdermal delivery. A nano-drug-delivery system can achieve sustained release of drugs. Therefore, the present disclosure designs and synthesizes a microneedle patch, by integrating the nano-drug delivery and the transdermal delivery, simultaneously realizing intracellular delivery of 5-ALA and catalase (CAT), thereby achieving actual effects of upregulating physiological synthesis concentration of PpIX, prolonging tumor-retention duration of PpIX, and resulting in improved therapeutic efficiency of 5-ALA-PDT.

Generation of high concentration of intratumoral singlet oxygen (¹O₂) is a key to a therapeutic effect of 5-ALA-PDT. However, adverse events of clinical trials of 5-ALA-PDT, such as pain, edema, and decrustation of tissues adjacent to a treatment site, are associated with high accumulation of ¹O₂ in normal tissues. This is due to the fact that ¹O₂ generated during a PDT reaction can trigger oxidative damage and secondary modification of proteins and polynucleotides, thereby making a membrane organelle more fragile and further inducing apoptosis or necrosis. In order to enhance anti-tumor efficiency of 5-ALA-PDT and reduce toxic and side effects, a more-accurate diagnosis-and-treatment plan of 5-ALA-PDT is urgently needed, especially needing a real-time evaluation of PpIX and O₂ concentrations in tumor tissue, and non-invasive molecular imaging to formulate laser-irradiation parameters, so as to optimize treatment parameters.

Therefore, the prior art still needs to be improved and developed.

### BRIEF SUMMARY OF THE DISCLOSURE

According to the above-mentioned defects of the prior art, a purpose of the present disclosure is to provide a microneedle patch for enhancing accumulation of Protoporphyrin IX in a solid tumor and a preparation method therefor, aiming to solve the problem of tumor hypoxia, low concentration of synthesized PpIX, and low efficiency of solid-tumor treatment due to lack of specificity-delivery strategies in existing 5-ALA-PDT.

The technical solutions adopted by the present disclosure are as follows.

A method for preparing a microneedle patch for enhancing accumulation of PpIX in a solid tumor, includes steps of:
dissolving albumin into Dubos medium, and adding CuCl₂ and CaCl₂ for in-situ mineralization to obtain CCP nanoparticles;
loading CAT and 5-ALA onto a surface of the CCP nanoparticles by a one-step carbodiimide coupling method and an electrostatic-adsorption method, respectively, to obtain CCPCA nanoparticles; and
loading the CCPCA nanoparticles and microneedle-matrix solution into a microneedle mold, and after vacuum drying and demolding, obtaining the microneedle patch for enhancing accumulation of PpIX in a solid tumor.

Optionally, the step of dissolving albumin into Dubos medium, and adding CuCl₂ and CaCh for in-situ mineralization to obtain CCP nanoparticles, includes:
dissolving the albumin into the Dubos medium, then adding CuCh solution, mineralizing at 37 °C for 24 hours, then adding CaCh solution, and obtaining the CCP nanoparticles by further mineralization at 37 °C for 24 hours.

Optionally, the albumin may be human serum albumin (HSA) or bovine serum albumin (BSA).

Optionally, a concentration of the CuCl₂ solution is 0.1 M, a concentration of the CaCh solution is 1M, and volumes of the CuCh solution and the CaCh solution added are the same.

Optionally, the step of loading CAT and 5-ALA onto a surface of the CCP nanoparticles by a one-step carbodiimide coupling method and an electrostatic-adsorption method, respectively, to obtain CCPCA nanoparticles, includes:
dispersing the CCP nanoparticles in buffer to obtain CCP-nanoparticle solution;
adding 1-ethyl-(3-dimethylaminopropyl) carbodiimide hydrochloride (EDC·HCl) solution to the CCP-nanoparticle solution, and after stirring for a first predetermined time at room temperature and dark condition, adding N-hydroxysuccinimide solution for reacting;
adding the CAT into the system after reacting, and after stirring for a second predetermined time, obtaining CCPC nanoparticles; and
dispersing the CCPC nanoparticles in 5-ALA solution, and after stirring for a third predetermined time at room temperature and dark condition, obtaining the CCPCA nanoparticles.

Optionally, in the CCPCA nanoparticles, a mass ratio of the CAT to the CCP nanoparticles is 0 to 0.1, and a mass ratio of the 5-ALA to the CCP nanoparticles is 0 to 0.1.

Optionally, in the CCPCA nanoparticles, a drug loading of the CAT is 0 to 7.9%, and a drug loading of the 5-ALA is 0 to 6.8%.

Optionally, the step of loading the CCPCA nanoparticles and microneedle-matrix solution into a microneedle mold, and after vacuum drying and demolding, obtaining the microneedle patch for enhancing accumulation of PpIX in a solid tumor, includes:
providing the microneedle mold;
dispersing the CCPCA nanoparticles in water to obtain CCPCA-nanoparticle suspension; and
adding the CCPCA-nanoparticle suspension to the microneedle mold, vacuum drying, injecting the microneedle-matrix solution into the microneedle mold, and after vacuum drying and demolding, obtaining the microneedle patch for enhancing accumulation of PpIX in a solid tumor.

Optionally, the microneedle-matrix solution includes sodium hyaluronate and superactive hyaluronic acid, and a weight ratio of the sodium hyaluronate to the superactive hyaluronic acid is 1:(1-5).

A microneedle patch for enhancing accumulation of PpIX in a solid tumor, and the microneedle patch includes a patch and a plurality of microneedles arranged in an array located at the patch;
the patch is loaded with microneedle matrix;
the microneedles are loaded with CCPCA nanoparticles; and the CCPCA nanoparticles include CCP nanoparticles and CAT and 5-ALA both loaded on a surface of the CCP nanoparticles; and
the microneedle patch is prepared by the methods in the present disclosure.

Optionally, the microneedle matrix includes sodium hyaluronate and superactive hyaluronic acid, and a weight ratio of the sodium hyaluronate to the superactive hyaluronic acid is 1:(1-5).

The present disclosure has following beneficial effects:
1. The present disclosure realizes co-delivery ability of efficient and precise transdermal delivery of CAT and 5-ALA through the combination of nano-sustained release and microneedle delivery strategy. Under the condition of giving the same concentration of 5-ALA (10.8 µg mL⁻¹), content (141.4 ± 1.7 ng/10⁶ cells) of CCPCA nanoparticles inducing PpIX accumulation in A375 melanoma cells is respectively 1.9 times more than CCPA group (72.4 ± 7.7 ng/10⁶ cells) not loaded with CAT and 4.0 times more than free 5-ALA group (35.1 ± 3.3 ng/10⁶ cells). Compared with method of intravenous injection or simple microneedle delivery of 5-ALA, the MN-CCPCA microneedle patch based on nanodiagnostic design of the present disclosure can significantly increase accumulation of PpIX at a tumor site. The maximum fluorescence intensity of PpIX in subcutaneous U87MG glioblastoma is 5.3 times and 2.7 times more than that in tail intravenous injection group of 5-ALA and microneedle delivery group of 5-ALA, respectively. The maximum fluorescence intensity of PpIX in subcutaneous 4T1 breast cancer cell is 4.3 times and 3.2 times more than that in the tail intravenous injection group of 5-ALA and the microneedle delivery group of 5-ALA, respectively.
2. A strategy of the present disclosure to upregulate PpIX accumulation is to increase endogenous and exogenous PpIX synthesis, and to minimize efflux of PpIX in a cancer cell. MN-CCPCA microneedle patch can reverse tumor hypoxia, regulate PpIX-related synthase, and circumvent a negative reaction regulation process of PpIX. A molecular mechanism is that CAT catalyzes endogenous hydrogen peroxide to continuously increase O₂ level and inhibits HIF-1α expression, so as to reduce FECH expression, thereby limiting efflux of PpIX in mitochondria and triggering a negative-feedback effect of heme shortage, increasing expression of ALA synthase (ALAS), making exogenous 5-ALA delivered by CCPCA nanoparticles and endogenous 5-ALA synthesized by a cancer cell can be simultaneously converted into PpIX, which further leads to increased accumulation of PpIX in a cell.
3. The present disclosure successfully provides a microneedle patch for enhancing accumulation of Protoporphyrin IX in a solid tumor. By reversing tumor hypoxia, maximum physiological concentration of PpIX synthesis at the tumor site is increased, and duration for maximum accumulation peak of PpIX is further prolonged (maximum tumor-retention durations of PpIX in glioblastoma, melanoma, and breast cancer are extended to 12, 12, and 24 hours, respectively, which are significantly higher than 4 hours of free 5-ALA), thereby providing a longer therapeutic window, so that under a premise of one administration, it can be used for multiple repeated PDT.
4. The present disclosure can formulate individualized treatment plans for various superficial tumors (breast cancer, glioblastoma, and melanoma), and can provide real-time PpIX fluorescence imaging and photoacoustic blood-oxygen imaging guidance simultaneously with the help of fluorescence and photoacoustic instruments, thereby improving effectiveness of cancer treatment.

### BRIEF DESCRIPTION OF THE DRAWINGS

FIG. 1 is a schematic diagram of an application of a microneedle patch for enhancing accumulation of PpIX in a solid tumor in Embodiment 1.
In FIG. 2, a is a transmission electron-microscope image of CCPCANPs obtained in Embodiment 1, and b in FIG. 2 is a scanning electron-microscope image of the MN-CCPCA microneedle patch obtained in Embodiment 1.
In FIG. 3, a is a high-resolution scanning electron-microscope image of the MN-CCPCA microneedle patch before loading the CCPCA NPs in Embodiment 1, and b in FIG. 3 is a high-resolution scanning electron-microscope image of the MN-CCPCA microneedle patch after loading the CCPCA NPs in Embodiment 1.
FIG. 4 shows encapsulation efficiency and drug loading of 5-ALAin the CCPCA NPs obtained in Embodiment 1.
FIG. 5 is a thermogravimetric curve of CCPA and the CCPCA NPs obtained in Embodiment 1.
FIG. 6 shows a quantitative value of generated dissolved oxygen catalyzed by the CCPCA NPs of different concentrations (0 to 160 mg/mL) obtained in Embodiment 1 under a condition of 1 mM H₂O₂.
FIG. 7 is a graph of a comparison of catalytic kinetics of CAT in the CCPCA NPs obtained in Embodiment 1 and the same amount of CAT in vitro both respectively catalyzing 10 mM H₂O₂.
FIG. 8 is a quantification curve of Ca²⁺ (a) and Cu²⁺ (b) released by the CCPCA NPs obtained in Embodiment 1 degrading in neutral (pH=7.4) and weakly acidic (pH=6.0) conditions.
FIG. 9 is a test graph of mechanical performance of the MN-CCPCA microneedle patch obtained in Embodiment 1.
FIG. 10 is an image of hematoxylin-eosin staining of the MN-CCPCA microneedle patch obtained in Embodiment 1 penetrating through stratum corneum in mouse epidermis (a) and rat epidermis (b).
FIG. 11 shows a quantification value of accumulation of PpIX in a cell by different nanoparticles obtained in the present embodiments.
FIG. 12 shows a fluorescence semi-quantitative value of PpIX generated by different groups in A375 tumor cell under a condition of adding equal amount of 5-ALA in the present embodiments.
FIG. 13 shows an intracellular expression of related synthase of different groups in regulating PpIX synthesis in the present embodiments.
FIG. 14 shows a fluorescence semi-quantitative value of PpIX generated by different microneedle groups in vivo A375 melanoma obtained in the present embodiments.
FIG. 15 shows a real-time quantitative value of blood-oxygen saturation of different microneedle groups in vivo A375 melanoma obtained in the present embodiments.
FIG. 16 is a tumor-volume inhibition curve (a) and a tumor image (b) obtained by different microneedle groups in vivo A375 melanoma after using PDT obtained in the present embodiments.
FIG. 17 shows a fluorescence semi-quantitative value of PpIX generated by groups of different administration modes in vivo U87MG glioblastoma obtained in the present embodiments.
FIG. 18 is a tumor-volume inhibition curve (a) and a tumor image (b) obtained by groups of different administration modes in vivo U87MG glioblastoma after using PDT obtained in the present embodiments.
FIG. 19 shows a fluorescence semi-quantitative value of PpIX generated by groups of different administration modes in vivo 4T1 triple-negative breast tumor after using PDT obtained in the present embodiments.
FIG. 20 is a tumor-volume inhibition curve (a) and a tumor image (b) obtained by groups of different administration modes in vivo 4T1 triple-negative breast tumor after using PDT obtained in the present embodiments.

### DETAILED DESCRIPTION OF THE EMBODIMENTS

The present disclosure provides a microneedle patch for enhancing accumulation of PpIX in a solid tumor and a preparation method therefor. In order to make the purposes, technical schemes, and effects of the present disclosure clearer and explicit, the present disclosure is further described in detail below. It should be understood that the specific embodiments described herein are only used to explain the present disclosure, not to limit the present disclosure.

An embodiment of the present disclosure provides a method for preparing a microneedle patch for enhancing accumulation of PpIX in a solid tumor, which includes following steps:
S1: Dissolving albumin (as a template) into Dubos medium, adding CuCh and CaCl₂ for in-situ mineralization to obtain CCP nanoparticles (denoted as CCP NPs);
S2: loading CAT and 5-ALA onto a surface of the CCP nanoparticles by a one-step carbodiimide coupling method and an electrostatic-adsorption method respectively, to obtain CCPCA nanoparticles (denoted as CCPCANPs); and
S3: loading the CCPCA nanoparticles and microneedle matrix solution into a microneedle mold, and after vacuum drying and demolding, obtaining the microneedle patch (referred to as MN-CCPCA) for enhancing accumulation of PpIX in a solid tumor.

In the present embodiment, the MN-CCPCA has co-delivery capability of efficient and precise transdermal delivery of CAT and 5-ALA. The CCPCA NPs have good weakly acidic pH (6.0-7.4) degradation ability, and ability to catalyze intratumoral hydrogen peroxide (H₂O₂) to generate O₂. The MN-CCPCA can reverse tumor hypoxia to regulate PpIX-related synthase and avoid negative reaction regulation process of PpIX. A specific molecular mechanism is that CAT continuously increases endogenous O₂ level and inhibits HIF-1α expression, decreasing FECH expression, thereby limiting efflux of PpIX in mitochondria and triggering a negative-feedback effect of heme shortage, increasing ALAS expression, leading to increased accumulation of PpIX in the mitochondria. The MN-CCPCA in the present embodiment can increase intratumoral PpIX and O₂ levels through precise delivery of CAT and 5-ALA, while providing guidance for real-time dual-modality PpIX fluorescence imaging and photoacoustic blood-oxygen imaging, thereby optimizing 5-ALA-based PDT parameters, improving PDT efficiency for three different cancers (breast cancer, glioblastoma, and melanoma), and reducing side effects.

In step S1, in one implementation, the albumin may be a protein such as human serum albumin (HSA) or bovine serum albumin (BSA).

In one implementation, the step of dissolving albumin into Dubos medium, adding CuCh and CaCl₂ for in-situ mineralization to obtain CCP nanoparticles, includes:
Dissolving HSA in sugar-free Dubos medium, then adding CuCl₂ solution (solvent is deionized water), mineralizing at 37°C for 24 hours, then adding CaCl₂ solution (solvent is deionized water), mineralizing at 37°C for 24 hours to obtain CCP nanoparticles. HSA having negative charge tends to adsorb Ca²⁺ and Cu²⁺ centrally, leading to local supersaturation of ions and providing nucleation sites, doping of Cu²⁺ is beneficial for stabilizing nucleation of CCPCA nanoparticles and hindering transformation of calcium phosphate from amorphous state to crystalline state, thereby inducing in-situ mineralization and forming nanoscale CCP particles.

In one implementation, a concentration of the CuCl₂ solution is 0.1 M, a concentration of the CaCh solution is 1 M, and volumes of the CuCh solution and the CaCl₂ solution added are the same.

In step S2, in one implementation, the step of loading CAT and 5-ALA onto a surface of the CCP nanoparticles by a one-step carbodiimide coupling method and an electrostatic adsorption method respectively, to obtain CCPCAnanoparticles, includes:
Dispersing the CCP nanoparticles in a buffer (such as a PBS buffer, pH is adjusted to about 7.4) to obtain CCP nanoparticle solution;
Adding EDC·HCl solution (solvent may be dimethyl sulfoxide) to the CCP nanoparticle solution, and after stirring for a first predetermined time (1 to 2 hours) at room temperature and dark condition, adding N-hydroxysuccinimide solution (solvent may be dimethyl sulfoxide) for reaction (time may be 5 to 15 minutes, such as 10 minutes), then adding CAT solution (concentration is about 0.2 mg/mL, solvent is deionized water), after stirring for a second predetermined time (time may be 5 to 7 hours, such as 6 hours), obtaining CCPC nanoparticles;
Dispersing the CCPC nanoparticles in 5-ALA solution (5-ALA is dispersed in buffer, the buffer may be 0.1 mM PBS solution, pH is adjusted to about 7.0), after stirring for a third predetermined time (the time may be 1 to 3 hours, such as 2 hours) at room temperature and dark condition, obtaining the CCPCA nanoparticles.

In one implementation, in the CCPCA nanoparticles, a mass ratio of CAT to CCP nanoparticles is 0 to 0.1, and a mass ratio of 5-ALA to CCP nanoparticles is 0 to 0.1.

In one implementation, in the CCPCA nanoparticles, drug loading of CAT is 0 to 7.9%, and drug loading of 5-ALA is 0 to 6.8%.

In step S3, the CCPCA nanoparticles and the microneedle matrix solution are loaded into the microneedle mold using a two-step micromolding process. In one implementation, the step of loading the CCPCA nanoparticles and microneedle matrix solution into a microneedle mold, and after vacuum drying and demolding, obtaining the microneedle patch (referred to as MN-CCPCA) for enhancing accumulation of PpIX in a solid tumor, includes:
Providing a microneedle mold (a material of the microneedle mold may be PDMS);
Dispersing the CCPCA nanoparticles in water to obtain CCPCA-nanoparticle suspension; and
Adding the CCPCA-nanoparticle suspension into the microneedle mold for vacuum-drying, injecting the microneedle matrix solution into the microneedle mold, and after vacuum-drying and demolding, obtaining the microneedle patch for enhancing accumulation of PpIX in a solid tumor were obtained.

In one implementation, the microneedle matrix solution includes sodium hyaluronate (molecular weight may be, but not limited to, 5 kDa) and superactive hyaluronic acid (molecular weight may be, but not limited to, 100 kDa), a weight ratio of the sodium hyaluronate and the superactive hyaluronic acid is 1:(1-5).
An embodiment of the present disclosure provides a microneedle patch (MN-CCPCA) for enhancing accumulation of PpIX in a solid tumor, furthermore the microneedle patch includes: a patch and a plurality of microneedles arranged in an array on the patch;
The patch is loaded with microneedle matrix;
The microneedles are loaded with CCPCA nanoparticles; Furthermore the CCPCA nanoparticles include CCP nanoparticles and CAT and 5-ALA both loaded on a surface of the CCP nanoparticles; and
The microneedle patch is prepared by the method described in the present embodiments of the present disclosure.

In one implementation, the microneedle matrix includes sodium hyaluronate and superactive hyaluronic acid, and a weight ratio of the sodium hyaluronate to the superactive hyaluronic acid is 1:(1-5).

In the present embodiment, MN-CCPCA is mainly composed of 5-aminolevulinic acid (5-ALA), catalase (CAT), copper calcium phosphate nanoparticles with tumor acid degradation, superactive hyaluronic acid, and sodium hyaluronate. Regulating PpIX-related synthase can maximize enrichment of PpIX at tumor sites. The MN-CCPCA can continuously produce oxygen through catalyzing endogenous hydrogen peroxide by CAT, significantly reversing tumor hypoxia. A false signal of "hemoglobin deficiency" is released by downregulating HIF-1α and FECH, reducing a rate of PpIX synthesizing heme. Simultaneously, upregulating ALAS promotes synthesis of endogenous and exogenous 5-ALA, and increases accumulation of PpIX at tumor sites. Compared with pure microneedle delivery of 5-ALA, the combination of microneedle transdermal-drug delivery and nano-sustained-release system strategy extend maximum tumor-retention duration of PpIX in glioblastoma, melanoma, and breast cancer to 12, 12, and 24 hours respectively, and fluorescence intensity at a maximum enrichment time point increased to 2.2, 2.7, and 3.2 times, respectively. The MN-CCPCA can monitor tumor oxygen saturation and PpIX real-time metabolic kinetics in vivo through fluorescence imaging and photoacoustic imaging, and optimize treatment parameters of various superficial a solid tumor, achieving reproducible, efficient, and accurate PDT based on 5-ALA-PDT, and having great clinical-translation advantages.

The present disclosure is further described through following embodiments.

Nanoparticles with different compositions and functions used in the following embodiments are prepared as follows:
Step 1: Dissolving 100 mg of HSA in 10 mL of Dubos medium without glucose, then adding 100 µL of CuCh solution (concentration is 0.1 M, solvent is deionized water), sealing obtained system and mineralizing at 37 °C for 24 hours, then adding 100 µL of CaCh solution (concentration is 1 M, solvent is deionized water) into the system, mineralizing continuously at 37 °C for 24 hours, then separating and collecting CCP NPs by centrifugation (12000 rpm, 10 minutes).
Step 2: Dispersing 2 mg of CCP NPs in 2 mL of 0.1 mM PBS buffer (pH is adjusted to 7.4) to obtain CCP NPs solution, dissolving 2 mg of 1-ethyl-(3-dimethylaminopropyl) carbodiimide hydrochloride (EDC·HCl) directly in 100 µL of DMSO and added to the above CCP NPs solution, after stirring for 1 hour at room temperature and dark condition, dissolving 2 mg of N-hydroxysuccinimide (NHS) in 100 µL of DMSO and added to stirred-activated reaction solution, then adding 0.2 mg/mL of CAT for stirring for 6 hours, centrifuging a obtained product at 12,000 rpm for 10 minutes, and separating and collecting CCPC NPs.
Step 3: Dispersing 2 mg of the CCPC NPs in 2 mL of 0.1 mg/mL of 5-ALA solution (0.1 mM in PBS, pH is adjusted to 7.0), then stirring for 2 hours at room temperature and dark condition, separating and collecting CCPCA NPs by centrifugation (12,000 rpm, 10 minutes).

Meanwhile, under other identical conditions, only steps 1 and 3 are taken to obtain CCPANPs.

MN-CCP, MN-CCPC, MN-CCPA, MN-CCPCA used in following embodiments are prepared according to following steps:
Corresponding different nanoparticles (i.e., CCP NPs, CCPC NPs, CCPA NPs, and CCPCA NPs) are dispersed in 600 µL of deionized water, respectively, to obtain nanoparticle suspension, taking and adding 100 µL of the nanoparticle suspension (10 mg mL⁻¹) into each PDMS microneedle mold, and then placing in a vacuum-drying oven, after continuously vacuuming 5 times (each time for 3 minutes), injecting 800 µL of microneedle matrix solution (including sodium hyaluronate and superactive hyaluronic acid, a concentration of the superactive hyaluronic acid is 180mg/mL, and a weight ratio of the sodium hyaluronate and super-active hyaluronic acid is 1:5) into each PDMS microneedle mold, drying at 37 °C for 13 hours for demolding, and finally storing in a desiccator at room temperature and dark condition.

### Embodiment 1: Preparation of a microneedle patch for enhancing accumulation of PpIX in a solid tumor

100 mg of HSA was dissolved in 10 mL of Dubos medium without glucose, and then 100 µL of CuCh solution (concentration of 0.1 M, deionized water as solvent) was added. The system was sealed and mineralized at 37 °C for 24 hours. Then, 100 µL of CaCl₂ solution (concentration of 1 M, deionized water as solvent) was added to the system, and mineralization was continued at 37 °C for 24 hours. Then, CCP NPs were separated and collected by centrifugation (12,000 rpm, 10 minutes).

2 mg of the CCP NPs were dispersed in 2 mL of 0.1 mM of phosphate buffer (PBS, pH adjusted to 7.4) to obtain CCP NPs solution. 2 mg of EDC·HCl was directly dissolved in 100 µL of DMSO directly, and then added to the above CCP NPs solution. After stirring and activation at room temperature and dark condition for 1 hour, 2 mg of N-hydroxysuccinimide (NHS) was dissolved in 100 µL of DMSO, and then added to reaction solution after the stirring and activation to react 10 minutes. Then, CAT solution (concentration of 0.2 mg/mL, deionized water as solvent) was added and stirred for 6 hours. The obtained product was centrifuged at 12,000 rpm for 10 minutes, and CCPC NPs were separated and collected.

2 mg of the CCPC NPs were dispersed in 2 mL of 0.1 mg/mL of 5-ALA solution (0.1 mM of PBS solution, pH adjusted to 7.0), and then stirred for 2 hours at room temperature and dark condition. Then, CCPCA NPs were separated and collected by centrifugation (12,000 rpm, 10 minutes).

The CCPCANPs were dispersed in 600 µL of deionized water to obtain CCPCA NPs suspension. 100 µL of the CCPCANPs suspension (10 mg mL⁻¹) was taken to deposit on a surface of each PDMS microneedle mold. The device was placed in a vacuum dryer, after 5 times of consecutive vacuum pumping (3 minutes each time), 800 µL of microneedle matrix solution (including sodium hyaluronate and superactive hyaluronic acid, a concentration of the superactive hyaluronic acid was 180 mg/mL, a weight ratio of the sodium hyaluronate to the superactive hyaluronic acid was 1:5) was injected into the each PDMS microneedle mold, demolded after drying at 37 °C for 13 hours, and finally stored in a desiccator protected from light at room temperature.

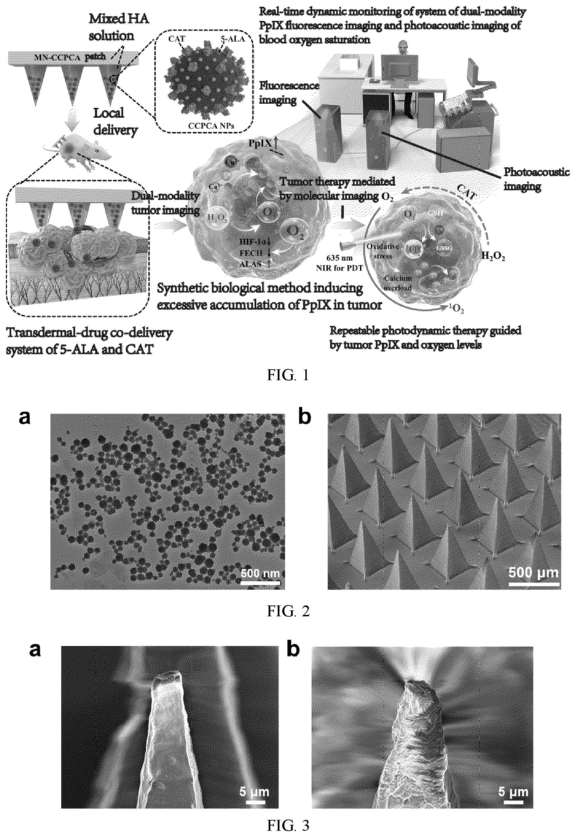

FIG. 1 is a schematic diagram of an application of a microneedle patch for enhancing accumulation of PpIX in a solid tumor in Embodiment 1. Specific application steps are as follows: pressing the prepared microneedle patch on a mouse tumor, removing the microneedle patch after applying 10 minutes, and using IVIS fluorescence-imaging spectroscopy system and Vevo LAZR-X photoacoustic imaging to monitor fluorescence intensity and blood-oxygen saturation (sO₂) of PpIX in tumor site in real time. Repeatable laser irradiation is performed according to the aforementioned two parameters, and tumor-volume changes are recorded (recorded every two days).

Combined with FIG. 1, the CAT significantly reverses tumor hypoxia by catalyzing endogenous hydrogen peroxide to continuously generate oxygen in vivo, releases a false signal of "hemoglobin deficiency" by downregulating HIF-1α and FECH to reduce a rate of PpIX synthesizing heme, while upregulates ALAS to promote synthesis of endogenous and exogenous 5-ALA, which further enhances accumulation of PpIX at tumor site. This MN-CCPCA can monitor in-vivo real-time PpIX metabolic kinetics and tumor oxygen saturation by PpIX fluorescence/photoacoustic imaging, while optimizes treatment parameters of various superficial solid tumors (breast cancer, glioblastoma, melanoma), so as to achieve repeatable, efficient, and accurate PDT based on 5-ALA-PDT.

In FIG. 2, a and b are a TEM image of the CCPCA NPs and a SEM image of the MN-CCPCA, respectively. It can be seen from the figure that the CCPCA NPs have good dispersion, and the microneedle array of the MN-CCPCA is uniformly arranged. Comparing the high-resolution scanning electron-microscope images of the CCPCA NPs before and after loading the microneedle in FIG. 3, it can be seen that the surface of the microneedle changed from smooth to uneven.

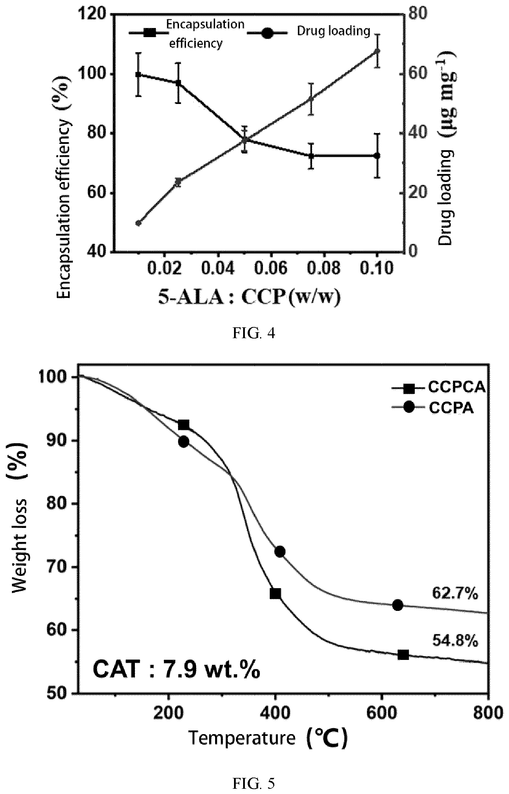

Then some basic performance of CCPCA NPs is studied. FIG. 4 shows the encapsulation efficiency and drug loading of 5-ALA in CCPCA NPs. When the mass ratio of 5-ALA to CCP is in the range of 0 to 0.1, the drug loading of 5-ALA is 0 to 6.8%. With the increase of the dosage ratio of 5-ALA, the drug loading increases accordingly. FIG. 5 shows thermogravimetric curves of CCPA and CCPCA NPs, indicating that when the mass ratio of CAT to CCP is in the range of 0 to 0.1, the drug loading of CAT is 0 to 7.9%. With the increase of the dosage ratio of CAT, the drug loading increases accordingly.

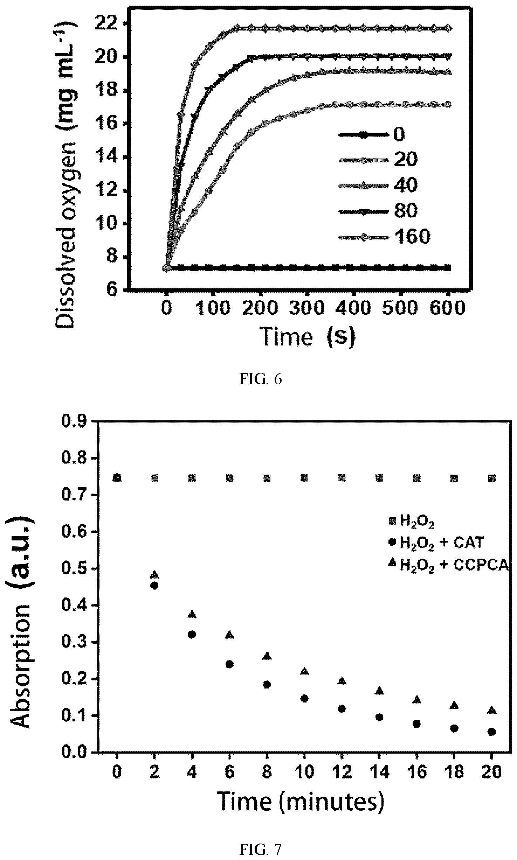

Then, the ability of CAT to catalyze H₂O₂ to generate O₂ in CCPCA NPs is verified. FIG. 6 shows the changes of different-concentration CCPCA NPs catalyzed H₂O₂ to generate dissolved oxygen with time under the condition of 1 mM of H₂O₂. FIG. 7 is a kinetic-comparison graph of CAT catalyzing 10 mM of H₂O₂ in CCPCANPs and the same amount of CAT catalyzing 10 mM of H₂O₂ in vitro, demonstrating that the activity of CAT in CCPCA NPs is equal to the activity of the same amount of free CAT.

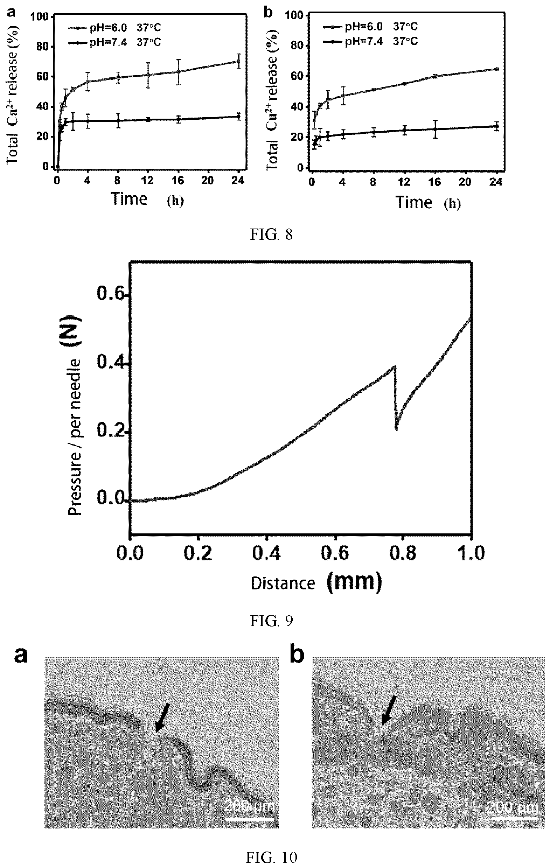

Then, the acid-responsive degradation ability of CCPCA NPs is verified. In FIG. 8, a and b are quantification curves of the obtained CCPCA NPs degraded in neutral (pH=7.4) and weakly acidic (pH=6.0) to release Ca²⁺ and Cu²⁺. Placing 1 mL of CCPCA NPs solution (concentration of 1 mg/mL, deionized water as solvent) in a dialysis bag, immersing in 9 mL of phosphate buffer with pH values of 6.0 and 7.4 respectively, taking 1 mL of solution at regular intervals for detection, and at the same time adding 1 mL of phosphate buffer corresponding to the pH value to the buffer. As shown in FIG. 8, CCPCA NPs has better Ca²⁺ and Cu²⁺ release effects at pH 6.0, showing excellent acid-responsive degradation performance.

The transdermal delivery ability of MN-CCPCA is studied. FIG. 9 is a graph of mechanical performance test of MN-CCPCA, showing that it can provide the transdermal penetration ability of 0.39 N/needle. FIG. 10 is an image of hematoxylin-eosin staining of the MN-CCPCA microneedle patch penetrating through stratum corneum in mouse epidermis (a) and rat epidermis (b), showing that MN-CCPCA can directly penetrate the stratum corneum of mice epidermis and rat epidermis, and achieve transdermal delivery of CAT and 5-ALA.

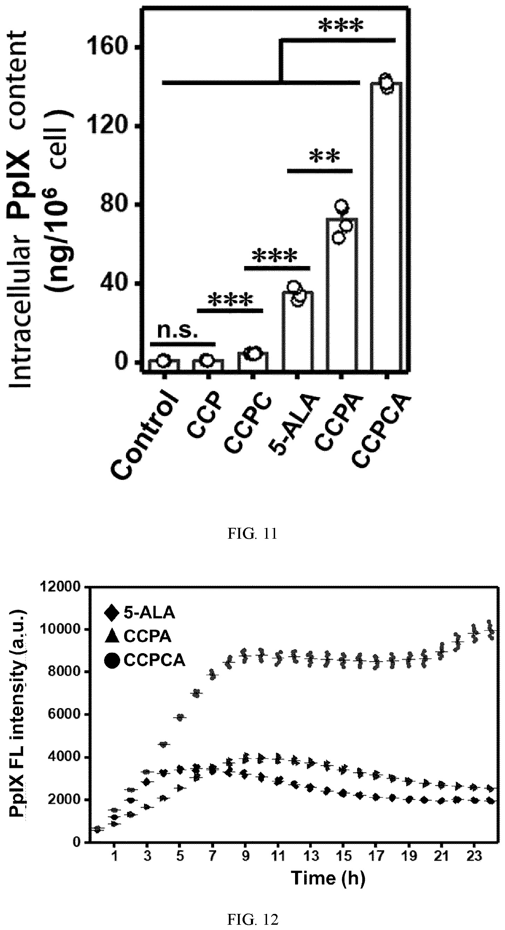

The biological effects of MN-CCPCA in accumulating PpIX in a cell and an in-vivo tumor are studied. FIG. 11 shows the mass comparison data of PpIX generated in A375 tumor cell in different groups. Under the condition of giving the same concentration of 5-ALA (10.8 µg mL⁻¹), the intracellular PpIX content in the CCPCA group (141.4±1.7 ng/10⁶ cells) is 1.9 times and 4.0 times more than that of CCPA without CAT (72.4±7.7 ng/10⁶ cells) and the free 5-ALA group (35.1±3.3 ng/10⁶ cells), respectively.

The dynamic fluorescence value of PpIX generated by an in-vivo cell is monitored in real time by high-content fluorescence microscopy. As shown in FIG. 12, the concentrations of PpIX in the 5-ALA group and the CCPA group reach the maximum at 6 and 10 hours after hatching, respectively, and then gradually decrease with time. The CCPCA group has the highest PpIX intensity at 24 hours, showing that the nanocarrier-based design extends the exposure time of 5-ALA in MN-CCPCA, and the PpIX retention time in the cell is also prolonged.

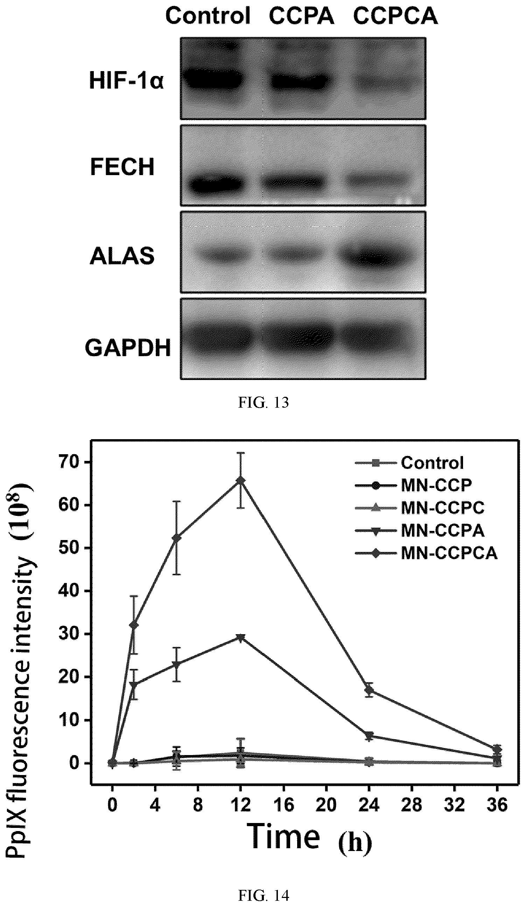

FIG. 13 shows the Western blot expression of the CCPCA group regulating the expression of PpIX-related synthase. MN-CCPCA can reverse tumor hypoxia to regulate PpIX-related synthase, and avoid the negative-reaction regulation process of PpIX. The molecular mechanism is that CAT catalyzes cell endogenous hydrogen peroxide continuously to increase O₂ level, inhibits HIF-1α expression, to reduce FECH expression, thereby limiting the efflux of PpIX in mitochondria and triggering a negative-feedback effect of heme shortage, increasing ALAS expression, leading to increasing accumulation of PpIX in cell.

FIG. 14 is a fluorescence quantitative value of accumulation of PpIX at different time points of intratumoral microneedles designed based on different nanocarriers, the microneedles are tracked by the IVIS spectroscopy system, and the microneedle patch for enhancing accumulation of PpIX in a solid tumor is in the treatment of a mouse A375 subcutaneous tumor model in Embodiment 1. The PpIX maximum fluorescence intensity of the MN-CCPCA in the A375 subcutaneous tumor model is 2.2 times more than that of the MN-CCPA group without CAT, demonstrating that simultaneous delivery of CAT and 5-ALA can significantly prolong the synthesis efficiency and tumor-retention duration of PpIX.

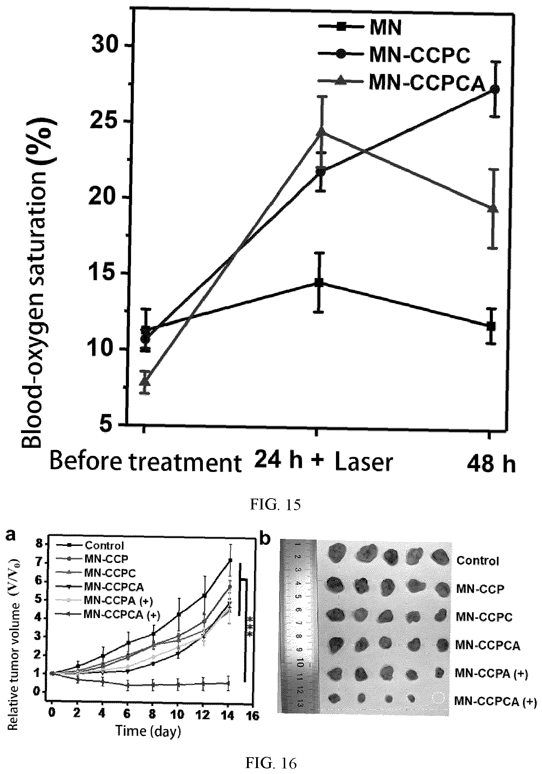

FIG. 15 is a quantitative value of the accumulation of blood-oxygen saturation (sO₂) at different times of administration tracked by a photoacoustic system, and the microneedle patch for enhancing accumulation of PpIX in a solid tumor is in the treatment of a mouse A375 subcutaneous tumor model in Embodiment 1. Compared with the blank microneedle control group, the MN-CCPCA increases the sO₂ level in the tumor by 2.3 times within 24 hours. Even after 24 hours of laser irradiation to consume oxygen, the sO₂ level of the MN-CCPCA group at 48 hours is still 1.68 times more than that of the control group.

FIG. 16 is a comparison image of tumor size and a comparison curve of tumor-volume inhibition obtained by 3 times of repeated PDT, and the microneedle patch for enhancing accumulation of PpIX in a solid tumor is in the treatment of a mouse A375 subcutaneous tumor model in Embodiment 1. A specific characterization method is as follows: 30 A375 model tumor mice with a tumor size of 70 mm³ are equally divided into 6 experimental groups, namely the blank microneedle group, the MN-CCP group not loaded with CAT and 5-ALA, the MN-CCPC group loaded with CAT, the MN-CCPCA group loaded with CAT and 5-ALA simultaneously, the MN-CCPA (+) group irradiated with laser and not loaded with CAT, the MN-CCPCA (+) group loaded with CAT and 5-ALA simultaneously and irradiated with laser. The doses administered are 50 mg/kg of CCPCANPs and 3.4 mg/kg of 5-ALA (each microneedle patch). The mice in the laser treatment group are irradiated with a laser with a wavelength of 635 nm for 10 minutes (200 mW/cm²) at 6, 12, and 24 h, respectively. The volume of mouse tumor in each group on different days (0, 2, 4, 6, 8, 10, 12, 14) is measured by a vernier caliper. As shown in FIG. 15, it can be seen from the changes in tumor volume that the tumor volume of the mice in the MN-CCPCA (+) group is significantly reduced, showing good PDT efficiency, demonstrating that simultaneously increasing the levels of PpIX and O₂ in tumor can greatly increase PDT efficiency.

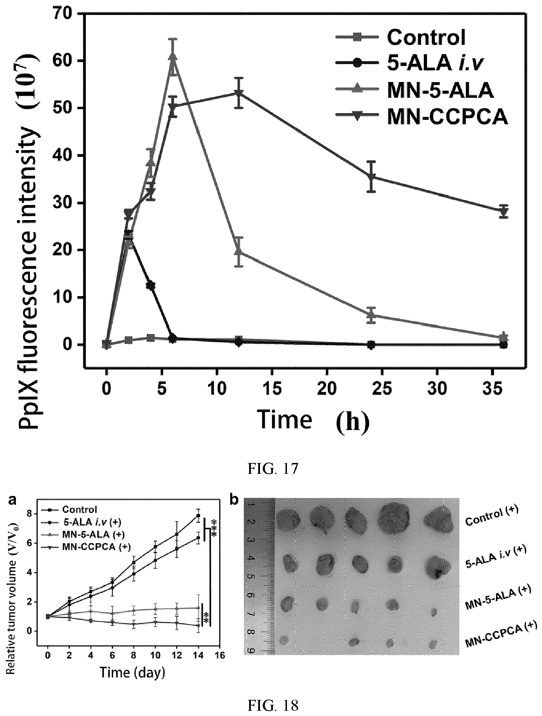

FIG. 17 is fluorescence quantitation of accumulation of PpIX at different time points of intratumoral different administration methods tracked by the IVIS spectroscopy system, and the microneedle patch for enhancing accumulation of PpIX in a solid tumor is in the treatment of a mouse subcutaneous U87MG glioblastoma model in Embodiment 1. The maximum fluorescence intensity of PpIX in the subcutaneous U87 glioblastoma of the MN-CCPCA is 5.3 times and 2.7 times more than that of the tail intravenous injection 5-ALA group and the microneedle delivery 5-ALA group, respectively.

FIG. 18 is a comparison image of tumor size and a comparison curve of tumor-volume inhibition obtained by 3 times of repeated PDT through improved 5-ALA delivery method, and the microneedle patch for enhancing accumulation of PpIX in a solid tumor is in the treatment of a mouse subcutaneous U87MG glioblastoma model in Embodiment 1. A specific characterization method is as follows: 20 U87MG model tumor mice with a tumor size of 100 mm³ are equally divided into 4 experimental groups, namely the MN (+) group with blank microneedle and irradiated with laser, the 5-ALAi.v (+) group with tail intravenous injected with 5-ALAand irradiated with laser, the MN-5-ALA (+) group with 5-ALA microneedle delivery and irradiated with laser, the MN-CCPCA (+) group with transdermal microneedle loaded with 5-ALA and CAT using nanoparticles and irradiated with lase. The doses administered are 50 mg/kg of CCPCA NPs and 3.4 mg/kg of 5-ALA. The mice in the laser treatment group are irradiated with a laser with a wavelength of 635 nm for 10 minutes (200 mW/cm²) at 6, 12, and 24 h, respectively. The volume of mouse tumor in each group on different days (0, 2, 4, 6, 8, 10, 12, 14) is measured by a vernier caliper. As shown in FIG. 15, it can be calculated from the changes in tumor volume that the tumor growth inhibition rates of 5-ALA i.v (+) group, MN-5-ALA (+) group, and MN-CCPCA (+) group are 19.1%, 79.7%, and 95.2%, the tumor volume of mice in the MN-CCPCA (+) group is significantly reduced, showing good PDT efficiency, indicating that the improved 5-ALA delivery strategy can greatly increase the PDT efficiency.

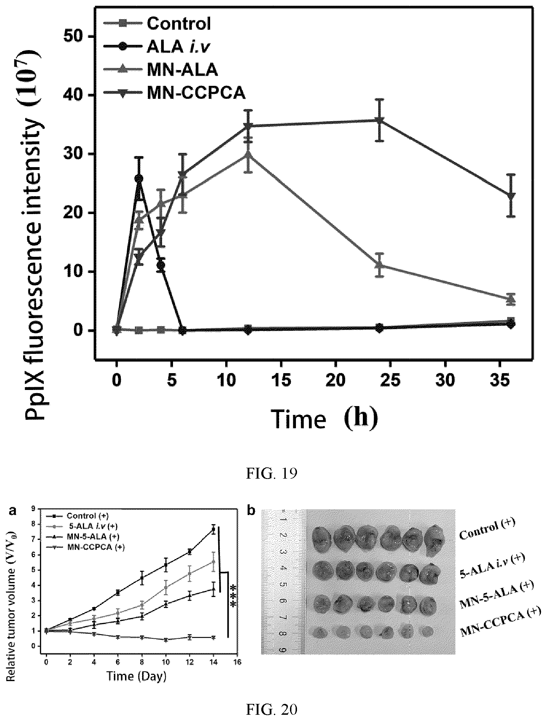

FIG. 19 is fluorescence quantitation of accumulation of PpIX at different time points of intratumoral different administration methods tracked by the IVIS spectroscopy system, and the microneedle patch for enhancing accumulation of PpIX in a solid tumor is in the treatment of a mouse 4T1 breast cancer subcutaneous tumor model in Embodiment 1. The maximum fluorescence intensity of PpIX in the 4T1 breast cancer subcutaneous tumor of the MN-CCPCA is 4.3 times and 3.2 times more than that of the tail intravenous injection 5-ALA group and the microneedle delivery 5-ALA group, respectively.

FIG. 20 is a comparison image of tumor size and a comparison curve of tumor-volume inhibition obtained by PDT through improved 5-ALA delivery method, and the microneedle patch for enhancing accumulation of PpIX in a solid tumor is in the treatment of a mouse 4T1 breast cancer subcutaneous tumor model in Embodiment 1. A specific characterization method is as follows: 20 4T1 model tumor mice with a tumor size of 100 mm³ are equally divided into 4 experimental groups, namely the MN (+) group with blank microneedle and irradiated with laser, the 5-ALA i.v (+) group with tail intravenous injected with 5-ALA and irradiated with laser, the MN-5-ALA (+) group with 5-ALA microneedle delivery and irradiated with laser, the MN-CCPCA (+) group with transdermal microneedle loaded with 5-ALA and CAT using nanoparticles and irradiated with lase. The mice in the laser treatment group are irradiated with a laser with a wavelength of 635 nm for 10 minutes (200 mW/cm²) at 12, 24, and 36 h, respectively. The doses administered are 50 mg/kg of CCPCANPs and 3.4 mg/kg of 5-ALA. The volume of mouse tumor in each group on different days (0, 2, 4, 6, 8, 10, 12, 14) is measured by a vernier caliper. As shown in FIG. 20, it can be calculated from the changes in tumor volume that the tumor growth inhibition rates of 5-ALA i.v (+) group, MN-5-ALA (+) group, and MN-CCPCA (+) group are 27.5%, 51.1%, and 92.3%, the tumor volume of mice in the MN-CCPCA (+) group is significantly reduced, showing good PDT efficiency, indicating that the improved 5-ALA delivery strategy can greatly increase the PDT efficiency.

It should be understood that the applications of the present disclosure are not limited to the above embodiments. For those ordinary skilled in the art, modifications or transformations can be made according to the above descriptions, and all these modifications and transformations should belong to the protection scope of the appended claims of the present disclosure.

## Claims

1. A method for preparing a microneedle patch for enhancing accumulation of PpIX in a solid tumor, comprising steps of:
dissolving albumin into Dubos medium, and adding CuCh and CaCl₂ for in-situ mineralization to obtain CCP nanoparticles;
loading CAT and 5-ALA onto a surface of the CCP nanoparticles by a one-step carbodiimide coupling method and an electrostatic-adsorption method, respectively, to obtain CCPCA nanoparticles; and
loading the CCPCA nanoparticles and microneedle-matrix solution into a microneedle mold, and after vacuum drying and demolding, obtaining the microneedle patch for enhancing accumulation of PpIX in a solid tumor.

2. The method for preparing a microneedle patch for enhancing accumulation of PpIX in a solid tumor according to claim 1, wherein the step of dissolving albumin into Dubos medium, and adding CuCl₂ and CaCl₂ for in-situ mineralization to obtain CCP nanoparticles, comprises:
dissolving the albumin into the Dubos medium, then adding CuCl₂ solution,
mineralizing at 37 °C for 24 hours, then adding CaCh solution, and obtaining the CCP nanoparticles by further mineralization at 37 °C for 24 hours.

3. The method for preparing a microneedle patch for enhancing accumulation of PpIX in a solid tumor according to claim 2, wherein the albumin is human serum albumin or bovine serum albumin, a concentration of the CuCl₂ solution is 0.1 M, a concentration of the CaCl₂ solution is 1M, and volumes of the CuCl₂ solution and the CaCl₂ solution added are the same.

4. The method for preparing a microneedle patch for enhancing accumulation of PpIX in a solid tumor according to claim 1, wherein the step of loading CAT and 5-ALA onto a surface of the CCP nanoparticles by a one-step carbodiimide coupling method and an electrostatic-adsorption method, respectively, to obtain CCPCA nanoparticles, comprises:
dispersing the CCP nanoparticles in buffer to obtain CCP-nanoparticle solution;
adding 1-ethyl-(3-dimethylaminopropyl) carbodiimide hydrochloride solution to the CCP-nanoparticle solution, and after stirring for a first predetermined time at room temperature and dark condition, adding N-hydroxysuccinimide solution for reacting;
adding the CAT into the system after reacting, and after stirring for a second predetermined time, obtaining CCPC nanoparticles; and
dispersing the CCPC nanoparticles in 5-ALA solution, and after stirring for a third predetermined time at room temperature and dark condition, obtaining the CCPCA nanoparticles.

5. The method for preparing a microneedle patch for enhancing accumulation of PpIX in a solid tumor according to claim 1, wherein in the CCPCA nanoparticles, a mass ratio of the CAT to the CCP nanoparticles is 0 to 0.1, and a mass ratio of the 5-ALA to the CCP nanoparticles is 0 to 0.1.

6. The method for preparing a microneedle patch for enhancing accumulation of PpIX in a solid tumor according to claim 1, wherein in the CCPCA nanoparticles, a drug loading of the CAT is 0 to 7.9%, and a drug loading of the 5-ALA is 0 to 6.8%.

7. The method for preparing a microneedle patch for enhancing accumulation of PpIX in a solid tumor according to claim 1, wherein the step of loading the CCPCA nanoparticles and microneedle-matrix solution into a microneedle mold, and after vacuum drying and demolding, obtaining the microneedle patch for enhancing accumulation of PpIX in a solid tumor, comprises:
providing the microneedle mold;
dispersing the CCPCA nanoparticles in water to obtain CCPCA-nanoparticle suspension; and
adding the CCPCA-nanoparticle suspension to the microneedle mold, vacuum drying,
injecting the microneedle-matrix solution into the microneedle mold, and after vacuum drying and demolding, obtaining the microneedle patch for enhancing accumulation of PpIX in a solid tumor.

8. The method for preparing a microneedle patch for enhancing accumulation of PpIX in a solid tumor according to claim 1, wherein the microneedle-matrix solution comprises sodium hyaluronate and superactive hyaluronic acid, and a weight ratio of the sodium hyaluronate to the superactive hyaluronic acid is 1:(1-5).

9. A microneedle patch for enhancing accumulation of PpIX in a solid tumor, wherein the microneedle patch comprises a patch and a plurality of microneedles arranged in an array located at the patch;
the patch is loaded with microneedle matrix;
the microneedles are loaded with CCPCA nanoparticles; wherein the CCPCA nanoparticles comprise CCP nanoparticles and CAT and 5-ALA both loaded on a surface of the CCP nanoparticles; and
the microneedle patch is prepared by the method according to any one of claims 1 to 8.

10. The microneedle patch for enhancing accumulation of PpIX in a solid tumor according to claim 9, wherein the microneedle matrix comprises sodium hyaluronate and superactive hyaluronic acid, and a weight ratio of the sodium hyaluronate to the superactive hyaluronic acid is 1:(1-5).
